# EUROPEAN PATENT SPECIFICATION

(11) **EP 1 711 212 B1**
(45) Date of publication and mention of the grant of the patent: **30.04.2008**
(21) Application number: 05702099.2
(22) Date of filing: 02.02.2005
(51) Int. Cl.: A61L 15/26, A61L 15/42

(54) **MEDICATED POLYURETHANE FOAMS**
MEDIZINISCHE POLYURETHANSCHAUMSTOFFE
MOUSSE DE POLYURETHANE MEDICAMENTEUSE

(30) Priority: 03.02.2004 GB 0402350
(43) Date of publication of application: 18.10.2006
(73) Proprietor: ETHICON INC., Somerville, NJ 08876 (US)
(72) Inventor: ADDISON, Deborah, Lancaster, LA2 8HB (GB); ESSLER, Alicia, Skipton,North Yorkshire BD23 2QX (GB); LAMB, Bruce, Richboro, PA 18754 (US)
(74) Representative: James, Anthony Christopher W.P.
(86) International application number: PCT/GB2005/000359
(87) International publication number: WO 2005/075001

(56) References cited:
- EP-A- 0 541 391
- EP-A- 1 112 739
- WO-A-00/47241
- WO-A-02/45761
- US-A- 4 339 550
- US-A- 5 776 495
- US-A- 5 833 665
- US-A1- 2002 168 400

## Description

This invention relates to medicated polyurethane foams, and more particularly to a method of making medicated polyurethane foams. The invention also relates to a wound dressing having a wound-contacting layer formed from such a foam.

Polyurethane foams have been proposed for a number of medicinal uses. The foams are prepared by reacting particular diisocyanates or isocyanate-capped prepolymers with suitable chain extending compounds having amine and/or alcohol multiple functionality. Chain terminating compounds such as mono-amines or monohydric alcohols may be included in the reaction mixture. Water may be included in the reaction mixture, since it reacts with isocyanate to liberate carbon dioxide for foaming the mixture.

US-A-4339550 discloses a hydrophilic foam composition which is prepared by the "in situ" reaction of an isocyanate-capped polyether prepolymer having a functionality of from about 2 to about 8, water, and a chemically compatible, essentially non-polar, volatile organic compound. The foam is stated to be capable of achieving a sustained, controlled release of the volatile materials from the foamed structure. Suitable "control release" ingredients include polyols, such as propylene glycol and glycerine.

EP-A-0541391 describes a method of forming a polyurethane foam suitable for use as a wound-contacting layer, the method comprising mixing 1 part by weight of an isocyanate-capped prepolymer having a relatively low isocyanate content of from 0.5 to 1.2 meq NCO groups/g with from 0.4 to 1.0 parts by weight of water in the presence of from 0.05 to 0.4 parts by weight of a C1 to C3 monohydric alcohol, and then drying the product. The use of a relatively small amount of water produces an initial reaction mixture of much higher initial viscosity. Carbon dioxide formed by hydrolysis of isocyanate end groups is therefore trapped, producing a foamed hydrogel. For use as a wound-contact layer, topical medicaments and antiseptics, such as silver sulfadiazine, povidone iodine, chlorhexidine acetate and chlorhexidine gluconate, as well as other therapeutically useful additives such as polypeptide growth factors and enzymes may be incorporated into one or more of the components used to make the foaming mixture.

US-A-5833665 describes medicated polysaccharide materials, wherein the polysaccharide has been cross-linked by treatment with an isocyanate prepolymer, and wherein the cross-linked polysaccharide has then been treated with a solution of a therapeutic agent and then dried. The polysaccharide provides a biodegradable, sustained release matrix for the therapeutic agent. Similar materials using a cross-linked protein matrix for the active agent are described in US-A-5002769. However, these materials based on cross-linked biopolymers are relatively expensive and can suffer from other drawbacks.

EP-A-1112739 describes formulations for sustained release of medicaments comprising a lipophilic drug and a water-soluble substance dispersed in a water-impermeable and biocompatible matrix material.

The present invention provides a method of forming a polyurethane foam suitable for use as a wound-contacting layer, said method comprising: mixing 1 part by weight of an isocyanate-capped prepolymer having from 0.5 to 1.2 meq NCO groups/g with from 0.4 to 1.0 parts by weight of water in the presence of from 0.05 to 0.4 parts by weight of a C 1 to C3 monohydric alcohol to form a foamed product; followed by treating the foamed product with a dispersion of a therapeutic agent and drying the treated foamed product.

It has been found, surprisingly, that polyurethane foams of this particular type that have been medicated by post-treatment with a dispersion of the medicament, exhibit sustained release behaviour superior to that of polyurethane foams that have been medicated by incorporating the medicament into the foaming polyurethane reaction mixture. The products made in accordance with the present invention exhibit sustained release behaviour comparable to that achieved with the prior art compositions containing a polysaccharide or protein matrix, but at lower cost.

The polyurethane foam is treated with the therapeutic agent after the step of forming the foam. That is to say, the therapeutic agent is not incorporated into one or more of the components making up the polyurethane reaction mixture. Instead, the foam is treated with a dispersion (that is to say, a suspension or solution in a suitable solvent) of the therapeutic agent after the foam formation and polyurethane curing reactions are substantially complete, preferably after substantially all of the isocyanate groups have reacted. In certain embodiments, the foam may be dried to remove residual water and alcohols before the step of treating with the therapeutic agent.

Foams produced according to the method of the invention typically have a density of at least 0.28 g/cm, and preferably at least 0.30 g/cm. Particularly preferred foams have a density in the range 0.32 to 0.48 g/cm, e.g. about 0.35 g/cm.

The foams produced according to the method of the invention also preferably have an elongation at break of at least 150%, and more preferably at least 300%. Particularly preferred foams according to the invention have an elongation at break in the range from 500 to 2000%.

The foams obtainable by the present invention are preferably free from biopolymers such as polysaccharides or polypeptides. In certain embodiments, the foams obtainable by the present invention consist essentially of polyurethane, the therapeutic agents, and optional plasticisers.

Depending on the proportions of other additives, the foams produced according to the method of the invention have an absorbency of at least 3 g saline/g, preferably at least 5 g/g, and more preferably from 8 to 20 g/g. The foams are thus highly absorbent, yet conformable.

The foams produced according to the method of the invention also have the property of swelling and expanding when water is absorbed. This is particularly advantageous in a wound contact layer, because the swelling of the foam causes it to move inwards towards the wound bed, thus filling the wound cavity. This encourages the wound to heal from the base upwards and outwards, and it discourages epithelialization over the wound surface before the bed has been filled with granulation tissue.

The degree of swelling of the foams produced according to the method of the present invention on complete saturation with an aqueous medium is typically at least 100% (expressed in terms of increase in volume), and preferably at least 200%. Preferred foams swell by 400 to 800%. Despite this high degree of swelling, however, the foams of the invention retain their integrity even after absorption of large quantities of water. Typically, the cells of the foams of the invention have an average diameter in the range 0.1 to 0.6 mm.

The prepolymer which is used in the method of the invention is preferably an isocyanate-capped polyether, such as an ethyleneoxy/propyleneoxy copolymer. A particularly suitable prepolymer is that available under Trade Mark HYPOL, Hydrogel.

Although the invention comprehends the use as the C1-C3 alcohol of any of methanol, ethanol or propanol, the use of methanol is particularly preferred. All three alcohols reduce the rate of reaction between the isocyanate-capped prepolymer and water, but the effect of methanol is more marked. A reduction of the reaction rate is desirable in order to facilitate mixing of the various components and spreading of -the reaction mixture into a layer of suitable thickness for curing.

It will be appreciated that other components may be added to the reaction mixture in the method of the invention, in order to give desired properties to the product. In particular, it is preferable to include a small proportion (e.g. up to 30% by weight of the wet composition) of a rubber, which may be either natural or synthetic. This has the effect of increasing the cure time for the polyurethane, and increases extensibility, strength and tack. Most importantly; it substantially reduces shrinkage of the gel on drying, and it also improves bubble formation, producing more regular, smaller bubbles.

Preferably, the rubber is added in the form of a latex, i.e. a suspension or emulsion of the rubber in an aqueous medium. The latex will generally comprise 40 to 70% solids by weight, e.g. 50 to 60% by weight. If the foam is to be used as a wound contact layer, the rubber must of course be pharmaceutically acceptable. Acrylic-based rubbers are particularly preferred. These are commercially available in the form of latexes, such as PRIMAL N-582 and RHOPLEX N-560, manufactured by the Rohm & Haas company.

In addition to the methanol or ethanol, other alcohols, and particularly polyols, may be included in the reaction mixture to produce a softer, more conformable foam. For example, a polyol sold by Bayer AG under the Trade Mark Levagel may be used. However, traces of such alcohols are likely to remain in the free form after the foaming reaction, and these traces may be difficult to remove from the foam merely by heating. The use of higher boiling alcohols is therefore preferably avoided if the foam is to be used as a wound contact layer, because of the likelihood that such alcohols will be leached from the foam during use of the dressing. When used as or in wound dressings, the foams of the invention preferably contain less than 1% by weight of water soluble alcohols, and more preferably less than 0.1% by weight.It is particularly preferred that the foams of the invention are essentially free of water soluble alcohols (eg. less than 0.01% by weight).

Especially suitable for treatment with a dispersion of a medicament in accordance with the present invention are the polyurethane foams available under the registered trade mark TIELLE from Johnson & Johnson Medical Ltd.

The therapeutic agent (medicament) may be antimicrobial drugs or macromolecules such as growth factors, antibacterial agents, antispasmodic agents, or any other active biological bioactive agent, such as adrenergic agents such as ephedrine, desoxyephedrine, phenylephrine, epinephrine and the like, cholinergic agents such as physostigmine, neostigmine and the like, antispasmodic agents such as atropine, methantheline, papaverine and the like, tranquilizers and muscle relaxants such as fluphenazine, chlorpromazine, triflupromazine, mephenesin, meprobamate and the like, antidepressants like amitriptyline, nortriptyline, and the like, antihistamines such as diphenhydramine, dimenhydrinate, tripelennamine, perphenazine, chlorprophenazine, chlorprophenpyradimine and the like, hyptotensive agents such as rauwolfia, reserpine and the like, cardioactive agents such as bendroflumethiazide, flumethiazide, chlorothiazide, aminotrate, propranolol, nadolol, procainamide and the like, angiotensin converting enzyme inhibitors such as captopril and enalapril, bronchodialators such as theophylline, steroids such as testosterone, prednisolone, and the like, antibacterial agents, e.g., sulfonamides such as sulfadiazine, sulfamerazine, sulfamethazine, sulfisoxazole and the like, antimalarials such as chloroquine and the like, antibiotics such as the tetracyclines, nystatin, streptomycin, cephradine and other cephalosporins, penicillin, semi-synthetic penicillins, griseofulvin and the like, sedatives such as chloral hydrate, phenobarbital and other barbiturates, glutethimide, antitubercular agents such as isoniazid and the like, analgesics such as aspirin, acetaminophen, phenylbutazone, propoxyphene, methadone, meperidine and the like, etc. These substances are frequently employed either as the free compound or in a salt form, e.g., acid addition salts, basic salts like alkali metal salts, etc. Simple antimicrobial compounds are preferred, in particular silver salts, povidone iodine, cadexomer iodine, triclosan, polyhexamethylene biguanide (PHMB), and chlorhexidine salts such as chlorhexidine gluconate (CHG).

Typically, the therapeutic agent dissolved or suspended in a suitable solvent such as water at a concentration typically of from about 0.01 % to about 20%w/v, for example from about 0.1 % to about 10wt%, will be contacted with the polyurethane foam by immersion. Suitable temperatures for the immersion are from about 0°C to about 80°C, for example from about 5°C to about 50°C. The foam is then removed from the solvent. It may be dried in air or other atmosphere, for example at a temperature of from about 20°C to about 80°C, or it may be freeze-dried. Preferably, the resulting material is sterilized, for example by gamma-irradiation.

The loading of the foam with the therapeutic agent may readily be determined based upon the weight of the solution taken up by the foam. Suitable loadings for antimicrobials such as chlorhexidine salts, povidone iodine or triclosan are from about 0.1wt% to about 10wt.%, for example from about 0.5wet% to about 5wt%, based on the dry weight of the foam.

In a preferred method for forming the loaded polyurethane foam, the therapeutic agent is dissolved in water at a suitable concentration, typically about 1-10% by weight, and the sponge is immersed therein for a period of about 10 to about 300 minutes at ambient temperature (about 20-25°C).

In a second aspect, the present invention provides a medicated polyurethane foam obtainable by a process according to the invention.

In a further aspect, the present invention also provides a wound dressing comprising a medicated polyurethane foam obtainable by a process according to the invention.

Suitably, the medicated polyurethane foam in the dressing is in the form of a sheet, for example of area about 1cm² to about 200cm², and suitably of uncompressed thickness about 1mm to about 5mm. The medicated polyurethane foam may preferably form the wound contacting layer of the wound dressing, but it could be any layer that is capable of fluid exchange with the wound surface.

Preferably, the wound dressing of the invention further comprises an absorbent layer and/or a backing layer. As will be evident from the above, the absorbent layer may, for example, be positioned intermediate the medicated polyurethane foam wound contacting layer from the backing layer. The area of the optional absorbent layer is typically in the range of from 1cm² to 200cm², more preferably from 4cm² to 100cm².

The optional absorbent layer may comprise any of the materials conventionally used for absorbing wound fluids, serum or blood in the wound healing art, including gauzes, nonwoven fabrics, superabsorbents, hydrogels and mixtures thereof. For example, the absorbent layer may be a nonwoven fibrous web, for example a carded web of viscose staple fibers. The basis weight of the absorbent layer may be in the range of 50-500g/m², such as 100-400g/m². The uncompressed thickness of the absorbent layer may be in the range of from 0.5mm to 10mm, such as 1mm to 4mm. The free (uncompressed) liquid absorbency measured for physiological saline may be in the range of 5 to 30 g/g at 25°. The viscose web may incorporate superabsorbent fibers, for example the product known as OASIS (registered trade mark)

Preferably, the wound dressing further comprises a backing layer covering the medicated polyurethane foam and the optional absorbent layer on the side opposite the wound-facing side of the dressing. The backing layer preferably provides a barrier to passage of microorganisms through the dressing and further preferably blocks the escape of wound fluid from the dressing. The backing layer may extend beyond at least one edge of the medicated polyurethane foam and optional absorbent layer to provide an adhesive-coated margin adjacent to the said edge for adhering the dressing to a surface, such as to the skin of a patient adjacent to the wound being treated. An adhesive-coated margin may extend around all sides of the medicated polyurethane foam and optional absorbent layer, so that the dressing is a so-called island dressing. However, it is not necessary for there to be any adhesive-coated margin.

Preferably, the backing layer is substantially liquid-impermeable. The backing sheet is preferably semipermeable. That is to say, the backing sheet is preferably permeable to water vapour, but not permeable to liquid water or wound exudate. Preferably, the backing sheet is also microorganism-impermeable. Suitable continuous conformable backing sheets will preferably have a moisture vapor transmission rate (MVTR) of the backing sheet alone of 300 to 5000 g/m²/24hrs, preferably 500 to 2000 g/m²/24hrs at 37.5 °C at 100% to 10% relative humidity difference. The backing sheet thickness is preferably in the range of 10 to 1000 micrometers, more preferably 100 to 500 micrometers.

Suitable polymers for forming the backing sheet include polyurethanes and poly alkoxyalkyl acrylates and methacrylates such as those disclosed in GB-A-1280631. Preferably, the backing sheet comprises a continuous layer of a high density blocked polyurethane foam that is predominantly closed-cell. A suitable backing sheet material is the polyurethane film available under the Registered Trade Mark ESTANE 5714F.

The adhesive layer (where present) should be moisture vapor transmitting and/or patterned to allow passage of water vapor therethrough. The adhesive layer is preferably a continuous moisture vapor transmitting, pressure-sensitive adhesive layer of the type conventionally used for island-type wound dressings, for example, a pressure sensitive adhesive based on acrylate ester copolymers, polyvinyl ethyl ether and polyurethane as described for example in GB-A-1280631. The basis weight of the adhesive layer is preferably 20 to 250 g/m², and more preferably 50 to 150 g/m². Polyurethane-based pressure sensitive adhesives are preferred.

Preferably, the adhesive layer extends outwardly from the absorbent layer and the medicated polyurethane foam to form an adhesive-coated margin on the backing sheet around the absorbent layer as in a conventional island dressing.

Preferably, the wound dressing according to the present invention is sterile and packaged in a microorganism-impermeable container.

An embodiment of the present invention will now be described further, by way of example, with reference to the accompanying drawing:

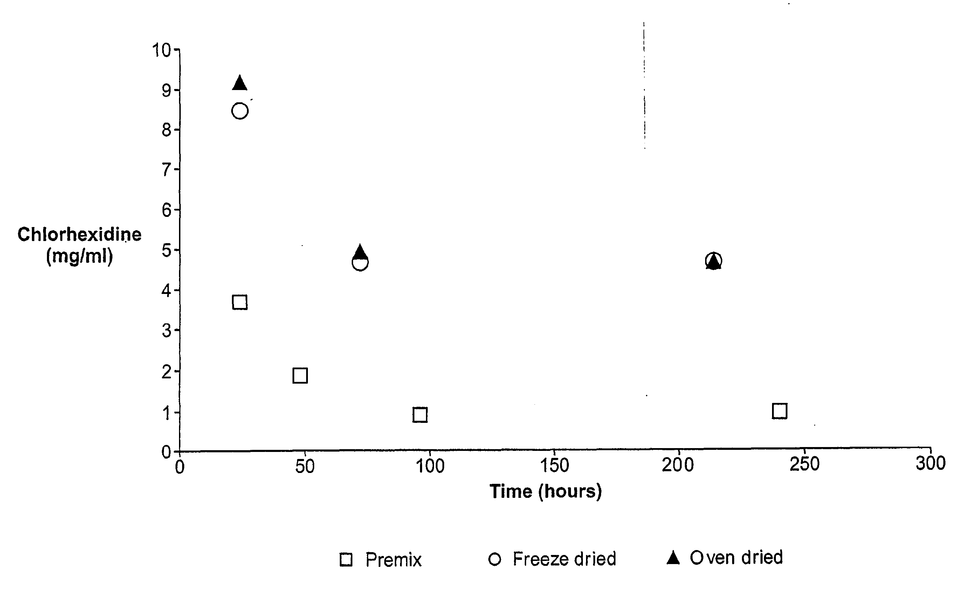

Figure 1 shows a graph of measured chlorhexidine gluconate release against time for two foamed materials made in accordance with the present invention (circles and triangles) and a comparative foamed material made by incorporating chlorhexidine gluconate into the polyurethane reaction mixture (squares).

### Example 1: Preparation of polyurethane foam

Methanol (6g) was added to HYPOL Hydrogel prepolymer (50g; NCO content 0.5-1.2 meq/g) in a disposable cup and mixed thoroughly for a few seconds. Water (44g) was then added to the HYPOL mixture and stirred vigorously. The foaming mixture was poured onto release paper and spread using a stainless steel hand spreader set at a gap of 2.2mm. The foam was left to cure and the foam sheet and release paper were placed in an oven (80-100°C) (30 min) to drive off the water. The resulting foam had a density of 0.38 g/cm, an elongation at break of 930%, and was capable of absorbing 10.7 g saline/g.

After cooling, the foam was lifted from the release paper, and immersed in an aqueous solution of chlorhexidine gluconate (10%w/v) for 30 minutes at room temperature, then lifted out. A first sample was then oven dried at 80- 100°C for 30 min. A second sample was blast frozen to -10°C and lyophilised (freeze dried).

Further samples were prepared from commercially available TTELLE (registered trade mark of Johnson & Johnson) polyurethane foams.

### Example 2 (Reference example)

In order to study the effect of different methods of incorporating the CHG, the procedure of Example 1 was followed except that the CHG was incorporated into the aqueous component of the foaming mixture at 2%w/v. No treatment of the foam product with aqueous CHG was carried out.

### Procedure 1

The sustained release of chlorhexidine from the foam materials of the invention was studied by placing a piece of foam in a reservoir of physiological saline solution at ambient temperature (20°C), with saline the reservoir changed daily. The CHG concentration in the solution was determined using UV/Visible spectrophotometer.

The results are shown in Figure 1. It can be seen that the medicated foams made by post-treatment with aqueous CHG followed by freeze drying (circles) or oven drying (triangles) give more sustained release of the CHG than the medicated foams made by incorporating CHG into the reaction mixture (squares). This result is surprising, since hitherto it has been conventional to incorporate actives into the reaction mixture of medical polyurethane foams.

The above embodiments have been described by way of example only. Many other embodiments falling within the scope of the accompanying claims will be apparent to the skilled reader.

## Claims

1. A method of forming a polyurethane foam suitable for use as a wound-contacting layer, said method comprising: mixing 1 part by weight of an isocyanate-capped prepolymer having from 0.5 to 12 meq NCO groups/g with from 0.4 to 1.0 parts by weight of water in the presence of from 0.05 to 0.4 parts by weight of a C1 to C3 monohydric alcohol to form a foamed product; followed by treating the foamed product with a dispersion of a therapeutic agent and drying the treated foamed product.

2. A method according to claim 1 wherein the monohydric alcohol is methanol.

3. A method according to claim 1 or claim 2 wherein the isocyanate-capped prepolymer is an isocyanate-capped polyether prepolymer.

4. A method according to claim 3 wherein the isocyanate-capped polyether prepolymer is an isocyanate-capped ethyleneoxy/propyleneoxy copolymer.

5. A method according to any preceding claim wherein one part by weight of the isocyanate-capped prepolymer is mixed with from 0.6 to 0.9 parts by weight of water.

6. A method according to any preceding claim wherein one part by weight of the isocyanate-capped prepolymer is mixed with water in the presence of from 0.05 to 0.25 parts by weight of methanol or from 0.1 to 0.3 parts by weight of ethanol.

7. A method according to any preceding claim wherein the method further comprises a step of drying the polyurethane foamed product before said step of treating the foamed product with a dispersion of a therapeutic agent.

8. A method according to any preceding claim wherein the foam is treated with an aqueous solution or suspension of the therapeutic agent having a concentration of from 1 to 20 wt.%, followed by drying or freeze drying.

9. A medicated polyurethane foam obtainable by a process according to any preceding claim.

10. A wound dressing comprising a medicated polyurethane foam according to claim 9.

## Patentansprüche

1. Verfahren zur Herstellung eines Polyurethanschaumstoffes, der zur Verwendung als eine mit einer Wunde in Kontakt kommende Lage geeignet ist, wobei das Verfahren umfasst: Vermischen von 1 Gewichtsteil eines mit Isocyanat überkappten Präpolymers mit 0,5 bis 1,2 mval NCO-Gruppen/g mit von 0,4 bis 1,0 Gewichtsteilen Wasser in Gegenwart von 0,05 bis 0,4 Gewichtsteilen eines einwertigen Cl- bis C3-Alkohols um ein geschäumtes Produkt zu bilden; gefolgt von der Behandlung des geschäumten Produktes mit einer Dispersion eines therapeutischen Mittels und Trocknen des behandelten geschäumten Produktes.

2. Verfahren nach Anspruch 1, **dadurch gekennzeichnet, dass** der einwertige Alkohol Methanol ist.

3. Verfahren nach Anspruch 1 oder Anspruch 2, **dadurch gekennzeichnet, dass** das mit Isocyanat überkappte Präpolymer ein mit Isocyanat überkapptes Polyether-Präpolymer ist.

4. Verfahren nach Anspruch 3, **dadurch gekennzeichnet, dass** das mit Isocyanat überkappte Polyether-Präpolymer ein mit Isocyanat überkapptes Ethylenoxy/Propylenoxy-Copolymer ist.

5. Verfahren nach einem vorangehenden Anspruch, **dadurch gekennzeichnet, dass** ein Gewichtsteil des mit Isocyanat überkappten Präpolymers mit von 0,6 bis 0,9 Gewichtsteilen Wasser vermischt wird.

6. Verfahren nach einem vorangehenden Anspruch, **dadurch gekennzeichnet, dass** ein Gewichtsteil des mit Isocyanat überkappten Präpolymers mit Wasser in Gegenwart von 0,05 bis 0,25 Gewichtsteilen Methanol oder von 0,1 bis 0,3 Gewichtsteilen Ethanol vermischt wird.

7. Verfahren nach einem vorangehenden Anspruch, **dadurch gekennzeichnet, dass** das Verfahren weiter den Schritt des Trocknens des geschäumten Polyurethan-Produktes vor dem Schritt der Behandlung des geschäumten Produktes mit einer Dispersion eines therapeutischen Mittels umfasst.

8. Verfahren nach einem vorangehenden Anspruch, **dadurch gekennzeichnet, dass** der Schaumstoff mit in einer wässrigen Lösung oder Suspension des therapeutischen Mittels mit einer Konzentration von 1 bis 20 Gew.-% behandelt wird, gefolgt von Trocknen oder Gefriertrocknen.

9. Medizinischer Polyurethanschaumstoff, der mit einem Verfahren nach einem vorangehenden Anspruch erhältlich ist.

10. Wundabdeckung, die einen medizinischen Polyurethanschaumstoff nach Anspruch 9 umfasst.

## Revendications

1. Procédé de formation d'une mousse de polyuréthane, pouvant être utilisée comme couche de contact avec une plaie, ledit procédé comprenant : le mélange d'1 partie en poids d'un prépolymère coiffé par un isocyanate ayant de 0, 5 à 1,2 meq de groupes NCO/gramme avec 0,4 à 1,0 partie en poids d'eau en présence de 0,05 à 0,4 partie en poids d'un alcool monohydrique en C₁ à C₃ pour former un produit expansé ; suivi d'un traitement du produit expansé avec une dispersion d'un agent thérapeutique et le séchage du produit expansé traité.

2. Procédé selon la revendication 1, dans lequel l'alcool monohydrique est le méthanol.

3. Procédé selon la revendication 1 ou 2, dans lequel le prépolymère coiffé par un isocyanate est un prépolymère de polyéther coiffé par un isocyanate.

4. Procédé selon la revendication 3, dans lequel le prépolymère de polyéther coiffé par un isocyanate est un copolymère d'éthylènoxy/propylènoxy coiffé par un isocyanate.

5. Procédé selon l'une quelconque des revendications précédentes, dans lequel une partie en poids du prépolymère coiffé par un isocyanate est mélangée avec 0, 6 à 0, 9 partie en poids d'eau.

6. Procédé selon l'une quelconque des revendications précédentes, dans lequel une partie en poids du prépolymère coiffé par un isocyanate est mélangée avec de l'eau en présence de 0,05 à 0,25 partie en poids de méthanol ou de 0,1 à 0,3 partie en poids d'éthanol.

7. Procédé selon l'une quelconque des revendications précédentes, dans lequel le procédé comprend en outre une étape de séchage du produit expansé de polyuréthane avant ladite étape de traitement du produit expansé avec une dispersion d'un agent thérapeutique.

8. Procédé selon l'une quelconque des revendications précédentes, dans lequel la mousse est traitée avec une solution ou suspension aqueuse de l'agent thérapeutique ayant une concentration allant de 1 à 20 % en poids, suivi par le séchage ou la lyophilisation.

9. Mousse de polyuréthane médicamenteuse pouvant être obtenue par un procédé selon l'une quelconque des revendications précédentes.

10. Pansement cicatrisant comprenant une mousse de polyuréthane médicamenteuse selon la revendication 9.
